Europäisches Patentamt

European Patent Office

Office européen des brevets

(11) Numéro de publication: **0 050 571**
**B1**

# FASCICULE DE BREVET EUROPEEN

(45) Date de publication du fascicule du brevet:
15.02.84

(51) Int. Cl.³: **C 12 N 1/16,** C 12 N 1/32 //
C12P39/00

(21) Numéro de dépôt: 81401640.8

(22) Date de dépôt: 19.10.81

(54) Procédé de culture de microorganismes, en particulier de levures, sur du lactoserum avec une association de souches judicieusement choisies et des mutants spécifiques d'une souche.

(30) Priorité: 20.10.80 FR 8022401

(43) Date de publication de la demande:
28.04.82 Bulletin 82/17

(45) Mention de la délivrance du brevet:
15.02.84 Bulletin 84/7

(84) Etats contractants désignés:
AT BE CH DE GB IT LI LU NL SE

(56) Documents cités:
CH - A - 233 548
FR - A - 1 002 238
FR - A - 1 128 063
FR - A - 2 300 803
FR - A - 2 439 819
US - A - 3 818 109

CHEMICAL ABSTRACTS, volume 91, no. 1, 2 juillet 1979,
page 383, abrégé 3927x COLUMBUS OHIO (US)

(73) Titulaire: **FROMAGERIES BEL, 4, rue d'Anjou,
F-75008 Paris (FR)**

(72) Inventeur: **Malige, Bernard, Les Persanges Saint Didier,
F-39000 Lons le Saunier (FR)**
Inventeur: **Goudal, Raymond, Résidence du
Parc 12 Faubourg Chartrain, F-41100 Vendôme (FR)**
Inventeur: **Moulin, Guy, 115 Chemin de l'Ere des
Masques Monferrier sur Lez, F-34980 Saint Gely du Fesc
(FR)**
Inventeur: **Galzy, Pierre, 1 Rue des Mélèzes,
F-34100 Montpellier (FR)**

(74) Mandataire: **Fruchard, Guy et al, NOVAPAT-CABINET
CHEREAU 107, boulevard Péreire, F-75017 Paris (FR)**

**Procédé de culture de microorganismes, en particulier de levures, sur du lactosérum avec une association de souches judicieusement choisies et des mutants spécifiques d'une souche**

La présente invention se se rapporte à l'industrie alimentaire et, plus spécifiquement, à un procédé d'optimisation de cultures de microorganismes, notamment de levures, spécialement de levures lactiques, pour la production de biomasse ou de métabolites intermédiaires de fermentation, utilisables dans l'industrie alimentaire.

Un des procédés connus de culture de levures lactiques sur du lactosérum est décrit dans le brevet français no 1.128.063 de la société dite Fromageries Bel. Il se rapporte à un procédé de fabrication de levures lactiques éclatées, qui consiste à séparer par précipitation les corps fermentescibles azotés, protéines et albumines, contenus dans le sérum de fromagerie ou de caséinerie ou dans le mélange sérum-babeurre, et à absorber et transformer par des levures les corps hydrocarbonés existant dans le sérum ou le mélange ainsi déprotéiné. La fermentation, menée dans des conditions de pH, d'aération et de températures précises, est effectuée par des souches de Saccaromyces, classées ultérieurement dans l'espèce Kluyveromyces (Lodder, 1970).

Depuis la date de dépôt (22 juin 1955) du brevet français no 1.128.063, ce procédé a fait l'objet de nombreuses améliorations, notamment en ce qui concerne l'adaptation des cultures aux nouvelles techniques de déprotéination du lactosérum, ainsi que le conditionnement et la conservation des cellules de levures obtenues. La déprotéination du lactose, autrefois effectuée par précipitation thermique, est maintenant réalisée par ultrafiltration et chromatographie échangeuse d'ions. De plus le substrat carboné, autrefois constitué de lactose, est maintenant formé de lactose hydrolysé, tel que cela est indiqué dans le brevet français no 2 439 819 déposé par la société dite des Fromageries Bel, où l'on conduit la culture d'un microorganisme sur le substrat laitier renfermant du lactose hydrolysé.

Actuellement, la production de levure-aliment est basée sur la culture préférentielle de certaines souches sur certains milieux: par exemple, le Kluyveromyces fragilis est cultivés sur du lactosérum, le Candida lipolytica sur des alcanes, le Candida tropicalis sur du gaz-oil et le Candida utilis sur des mélasses.

Ces fermentations, conduites le plus souvent dans des conditions de non-stérilité, entraînent la présence, dans le mélange, d'autres espèces qui, le plus souvent, ne sont pas souhaitées. De ce fait, un inconvénient résultant est que la souche souhaitée peut être éliminée du fermenteur par une souche «pirate contaminante».

De plus si l'on veut obtenir le développement d'une culture pure, il faut que le substrat carboné de croissance soit de qualité stérile constante pour maintenir une souche unique de fermentation. Or, les lactosérums provenant de la fabrication de fromages à caillé lactique ou de caséine lactique (lactosérums acides) contiennent 4 à 10 g/l d'acide lactique, alors que les lactosérums provenant de la fabrication de fromages à caillé présure ou de caséines-présure (lactosérums doux) ne contiennent que 1 à 3 g/l d'acide lactique. De plus, la variation de la teneur en acide lactique du substrat es due également au fait que les unités industrielles de traitement du lactosérum reçoivent cette matière à traiter continuellement tout le long de la journée et, en fonction de son origine, ce lactosérum a un rapport lactose/acide lactique qui varie tout le long de celle-ci.

Pour répondre à la variation de ce rapport, il est nécessaire de concevoir un système susceptible de s'adapter à ces variations par une sélection de souches présentant une spécificité d'assimilation des substrats. Une telle spécificité, compte tenu des phénomènes de diauxie existant en culture continue, n'est envisageable que par la sélection de mutants qui auront perdu la capacité d'assimiler l'un ou l'autre des substrats carbonés présents. On entend par diauxie, la capacité d'adaptation d'une souche à la consommation de différents substrats l'un aprés l'autre, ce qui impose le choix entre des vitesses de croissance limitée de façon à consommer toutes les sources de carbone ou des vitesses de croissance rapide et une perte de substances carbonées.

L'augmentation de la vitesse de croissance d'une culture dans laquelle il y a spécificité de consommation d'une espèce vis-à-vis d'un substrat se traduit en fait par la saturation du métabolisme oxydatif des Kluyveromyces et une partie de la source de carbone est dégradée par voie fermentaire, ce qui conduit à l'apparition obligatoire d'éthanol. Cet éthanol est connu comme étant un métabolite toxique pour les cellules; de ce fait, sa présence entraîne une inhibition de la croissance des levures. On se trouve dés lors limité par la concentration de ce métabolite dans ce milieu. Il est donc indispensable, pour imposer des vitesses de croissance maxima, d'éliminer ce métabolite; or, il représente une potentialité de synthèse. Une façon judicieuse de profiter de cette potentialité est d'introduire une souche capable d'assimiler ce substrat et ce de façon spécifique.

Il serait donc utile de remèdier aux inconvénients du procédé habituel et d'apporter une solution aux problèmes décrits ci-dessus. La présente invention a pour but de permettre l'optimisation des conditions de culture sur un substrat lactique, en se basant sur les techniques de culture actuellement connues et, notamment, sur l'opportunité d'avoir des cultures mixtes, tel que cela est décrit dans un article de Harrisson P.E. intitulé: «Cultures mixtes dans les procédés de fermentation industrielle» (Advances in Applied Microbiology (1978), 24, p. 129–164) qui donne un exemple de cultures mixtes sur du méthane et du méthanol. L'emploi des cultures mixtes permet une augmentation des performances par utilisation des différents substrats.

Un objet de la présente invention est de prévoir un procédé de culture avec association de souches, dont chacune utilisera un constituant différent du milieu, et ceci de façon exclusive, ce qui fournit une absence de compétition vis-à-vis des différents autres substrats du milieu.

Un autre objet de la présente invention est de sélectionner des mutants spécifiques d'une souche pour un substrat afin de créer des «niches» écologiques, chaque «niche» correspondant à une association de souches appartenant à la même espèce, souches qui seront dépendantes du développement global de la culture, donc des «niches» voisines.

D'autres objets apparaîtront d'après la description suivante.

Ces objets sont maintenant atteints par un procédé de culture de microorganismes, en particulier de levures, sur du lactosérum en tant que substrat de départ, procédé dans lequel, après séparation des protéines contenues dans le lactosérum et récupération des autres constituants, on réalise la culture sur le substrat lactique ainsi obtenu en utilisant, d'une part, une association de souches judicieusement choisies, dont chacune se développe d'une façon spécifique sur un substrat donné présent naturellement dans le milieu ou sur les métabolites formés au cours du processus de fermentation et, d'autre part, des mutants spécifiques d'une souche afin de créer des «niches» écologiques correspondant à une association de souches appartenant à la même espèce.

Dans la présente invention, les milieux de culture sont des lactosérums provenant de différents traitements laitiers et fromagers, c'est-à-dire des lactosérums doux ou acides, partiellement ou totalement déprotéinés par diférents procédés connus et utilisables industriellement (précipitation par traitement thermique, séparation par ultrafiltration, chromatographie d'exclusion et d'échange d'ions, etc.) et partiellement ou totalement hydrolysés par voie enzymatique (traitement avec une β-galactosidase) ou chimique.

Quel que soit le milieu de culture d'origine, il se compose toujours, d'une part, de lactose (ou d'un mélange de glucose, de galactose et de lactose dans le cas d'un lactose hydrolysé) et, d'autre part, d'acide lactique et d'autres acides organiques. La proportion de ces deux catégories de substrats peut varier selon l'origine et la technologie d'obtention du milieu.

Dans le procédé de la présente invention, on sélectionne judicieusement plusieurs souches à hautes performances en ce qui concerne leur taux de croissance ou le rendement de leur composition en protéines et leur spécificité à croître sur un substrat, et on les associe en vue de les cultiver sur chacun des substrats carbonés présents même en faible quantité dans la matière naturelle de départ ou sur les métabolites formés au cours du processus de fermentation. Au bout d'un certain temps, il résulte un équilibre de flore très stable, la proportion des différentes espèces dépendant de la concentration des substrat les uns par rapport aux autres.

Pour fonctionner de manière optimale, un tel système nécessite des «niches» écologiques. A l'intérieur de chacune d'entre elles, il existe une compétition entre les différentes souches susceptibles de croître avec les vitesses variables sur le seul substrat présent. Il est évident que les sélections à l'intérieur de chacune des «niches» sont dépendantes, en particulier pour la niche «éthanol», dont la concentration en substrat dépend de l'activité fermentaire des souches appartenant aux deux autres «niches» (lactose et ses dérivés d'hydrolyse acide lactique et autres acides organiques) et, en particulier, à la niche «lactose».

Les souches sélectionées appartiennent notamment aux espèces suivantes: Kluyveromyces fragilis, Kluyveromyces lactis et Torulopsis bovina. Les Kluyveromyces fragilis se développent sur du lactose, les Kluyveromyces lactis sur l'acide lactique, sans possibilité d'interférences, et les Torulopsis bovina sur l'éthanol. L'équilibre entre les souches varie selon la proportion des substrats dans le milieu et de la capacité du fermenteur pour la quantité d'éthanol produite.

A titre d'exemple, on peut donner la répartition suivante dans un milieu composé de lactose, d'acide lactique et d'éthanol comprenant 90-95% de lactosérum, 5-10% d'acide lactique et 1-3% d'éthanol:

- Kluyveromyces fragilis; environ 90 à 95%
- Kluyveromyces lactis; environ 5 à 10%
- Torulopsis bovina; environ 1 à 3%

Si le milieu est du lactosérum partiellement ou totalement hydrolysé, ce qui entraîne la présence de deux substrats carbonés supplémentaires, le glucose et le galactose, il peut être intéressant d'utiliser une nouvelle souche de levures telle que Saccharomyces cerevisae ou Candida utilis. On doit noter que le taux d'hydrolyse du lactose peut varier entre 0 et 100%, la répartition du glucose et du galactose entre 0 et 45% et celle du lactose entre 0 et 95%.

L'équilibre des souches, qui varie en fonction de la nature des substrats, est le suivant, lorsqu'il y a hydrolyse du lactose à 80-90% et que les substrats sont constitués de 10-20% de lactose, de 36-43% de glucose, de 36-43% de galactose, de 5-10% d'acide lactique et de 1-3% d'éthanol:

- Kluyveromyces fragilis; environ 45 à 65%
- Kluyveromyces lactis; environ 5 à 10%
- Candida utilis; environ 35 à 45%
- Torulopsis bovina; environ 1 à 3%

Les fermenteurs employés travaillent, dans des conditions optima, à des températures comprises entre environ 30 et 40°C, à un pH d'environ 3 à 4, avec un débit d'arrivée d'air égal à environ 1800 m³/h, avec une gamme d'environ 2 à 3 milliards de cellules totales par cm³, cette gamme étant la même quelle que soit la souche. On doit remarquer que, le volume d'air étant toujours fonction du volume du milieu, celui de 1800 m³/h correspond à un volume du milieu de 25 m³. En exprimant le volume d'air non pas en m³/h mais en VVm (volume d'air/volume du milieu/mn), les conditions optima se situent entre 1,2 et 1,5. Comme on travaille en continu, le débit est constant pendant la fermentation et avant le soutirage.

Le rendement en biomasse et/ou en métabolites peut être augmenté en incorporant dans le milieu de culture des substances minérales, telles que le phosphore, le cuivre, le zinc, le fer et divers autres éléments qui sont des substances nutritives pour les microorganismes (addition de $H_3PO_4, CuSO_4, ZnSO_4, FeSO_4$).

Par rapport aux procédés de culture existant actuellement, à une seule souche primordiale et à souches secondaires parasites, le procédé de la présente invention a les avantages suivants:

1. Il est possible d'adapter le procédé aux différents rapports lactose/acide lactique existant dans les lactosérums, qui sont les milieux naturels de culture.

2. On utilise la totalité de chaque source de carbone avec limitation des pertes, d'où l'obtention d'un rendement optimum, qui est d'environ 50 à 55%. En l'absence de souche métabolisant l'éthanol, la perte serait au moins égale à la proportion de Torulopsis bovina, soit 2 à 3%.

3. Il est possible de travailler avec des vitesses maxima de croissance des souches principales, du fait de la consommation par une tierce souche de métabolite intermédiaire toxique, d'où l'obtention d'une productivité maxima de l'ensemble du dispositif (fermenteur); le gain de productivité est environ 15 à 20% par rapport au procédé normal à une seule souche primordiale.

4. On supprime la compétitivité «interniches», tout en maintenant, d'une part, la compétitivité à l'intérieur de chaque niche et, d'autre part, la compétitivité des souches entre les niches.

5. Les installations ne sont pas sophistiquées en ce qui concerne l'aération et la limitation des transferts air-liquide qui constituent habituellement le facteur limitant ces installations.

6. La conduite des opérations est simplifiée.

Le procédé de la présente invention permet la production de biomasse (levure-aliment) et/ou de métabolites intermédiaires.

La présente invention sera maintenant décrite à l'aide des exemples suivants qui ne sont donnés qu'à titre d'illustration de la présente invention.

Exemple 1
On part d'un lactosérum doux provenant d'une fromagerie que l'on déprotéine par ultrafiltration.

Ce lactosérum déprotéiné est envoyé dans un fermenteur dit Airlift, travaillant dans les conditions suivantes:

– volume utile: 23 m³
– débit: 7600 l/h
– pH: 3 à 4

– température: 30 à 40°C
– débit d'air: 1800 m³/h
– addition d'une source azotée: $NH_4OH$

Les souches sélectionnées sont les suivantes, avec l'équilibre indiqué ci-dessous:

– Kluyveromyces fragilis sur lactose: 90–96%
– Kluyveromyces lactis sur acide lactique: 5–10%
 – Torulopsis bovina sur éthanol: 1 à 3%

Des contrôles systématiques sur deux ans de la flore présente dans le milieu ont montré une répartition très stable entre les espèces.

Exemple 2
On part d'un lactosérum acide provenant d'une caséinerie, que l'on a déprotéiné par ultrafiltration et dont on a hydrolysé partiellement (à 90%) le lactose à l'aide d'une β-galactosidase.

La fermentation se fait selon les mêmes conditions que celles données dans l'exemple 1.

Les souches sélectionnées sont les suivantes:

– Kluyveromyces fragilis sur lactose et galactose
– Kluyveromyces lactis sur acide lactique
– Candida utilis sur glucose
– Torulopsis bovina sur éthanol

La répartition des souches est la suivante:

– Kluyveromyces fragilis: 49,5–58,5%
– Kluyveromyces lactis: 5–10%
– Candida utilis: 40–43%
– Torulopsis bovina: 1–3%

Des contrôles systématiques sur deux ans de la flore présente dans le milieu ont montré une répartition très stable entre les espèces.

**Revendications**

1. Procédé de culture de microorganismes, en particulier de levures, sur du lactosérum en tant que substrat de départ, caractérisé en ce qu'après séparation des protéines contenues dans le lactosérum et récupération des autres constituants, on réalise la culture sur le substrat lactique ainsi obtenu, en utilisant, d'une part, une association de souches, dont chacune se développe d'une façon spécifique sur un substrat donné présent naturellement dans le milieu, ou sur les métabolites formés au cours du processus de fermentation, et, d'autre part, des mutants spécifiques d'une souche afin de créer des «niches» écologiques correspondant à une association de souches appartenant à la même espèce.

2. Procédé selon la revendication 1, caractérisé en ce que l'association de souches est constituée de Kluyveromyces fragilis, se développant sur du lactose, de Kluyveromyces lactis, se développant sur de l'acide lactique, de Candida utilis et de Saccharomyces cerevisae, se développant sur du glucose, et de Torulopsis bovina se développant sur de l'éthanol.

3. Procédé selon la revendication 1, caractérisé en ce que le substrat de départ est un lactosérum doux ou acide provenant de divers traitements laitiers et fromagers.

4. Procédé selon la revendication 3, caractérisé en ce que le substrat de départ est partiellement ou totalement déprotéiné par précipitation à l'aide d'un traitement thermique ou acide, par séparation par ultrafiltration ou chromatographie d'exclusion et d'échange d'ions.

5. Procédé selon la revendication 3, caractérisé en ce que le substrat de départ est également partiellement ou totalement hydrolysé par voie enzymatique ou chimique.

6. Procédé selon l'une quelconque des revendications 1 à 5, caractérisé en ce que, quelle que soit son origine, le milieu de culture est composé d'un mélange de deux (ou davantage) substrats choisis dans le groupe se composant de lactose, d'acide lactique, d'éthanol et, éventuellement, de galactose et de glucose.

7. Procédé selon la revendication 1, caractérisé en ce qu'à l'intérieur de chacune des «niches», on maintient une compétition entre les différentes souches susceptibles de croître avec le meilleur rendement sur le seul substrat présent.

8. Procédé selon la revendication 7, caractérisé en ce que les souches sélectionnées appartiennent aux espèces Kluyveromyces fragilis, Kluyveromyces lactis, Torulopsis bovina, Saccharomyces cerevisae ou Candida utilis.

9. Procédé selon l'une quelconque des revendications 1 à 8, caractérisé en ce que les souches sélectionnées sont des microorganismes qui ont l'aptitude à croître sur le ou les substrats, présents dans le milieu, qui se forment au cours du processus de fermentation.

## Claims

1. A method for culturing microorganisms, particularly yeasts, on lactoserum as starting substrate characterized in that, after separating proteins in lactoserum and recovering the other constituents, the culture is carried out on the thus obtained lactic substrate, by using, on one hand, an association of strains, each of which develops specifically on a given substrate present naturally in the medium, or on metabolites formed during the fermentation process, and, on the other hand, specific mutants of a strain so as to create ecological «niches» corresponding to an association of strains pertaining to the same species.

2. Method according to claim 1, characterized in that the association of strains consists of Kluyveromyces fragilis, developing on lactose, Kluyveromyces lactis, developing on lactic acid, Candida utilis and Saccharomyces cerevisae, developing on glucose, and Torulopsis bovina developing on ethanol.

3. Method according to claim 1, characterized in that the starting substrate is a mild or acidic lactoserum coming from various milk and cheese treatments.

4. Method according to claim 3, characterized in that the starting substrate is partially or wholly deproteinated by precipitation with the aid of a thermal or acidic treatment, by ultrafiltration separation or exclusion and ion exchange chromatography.

5. Method according to claim 3, characterized in that the starting substrate is also partially or wholly hydrolysed enzymatically or chemically.

6. Method according to any of claims 1 to 5, characterized in that, whatever its origin may be, the culture medium consists of a mixture of two or more substrates selected from the group consisting of lactose, lactic acid, ethanol and, possibly, galactose and glucose.

7. Method according to claim 1, characterized in that, inside each of the «niches», a competition is maintained between the different strains amenable to grow with the best yield on the single present substrate.

8. Method according to claim 7, characterized in that the selected strains pertain to Kluyveromyces fragilis, Kluyveromyces lactis, Torulopsis bovina, Saccaromyces cerevisae or Candida utilis species.

9. Method according to any of claims 1 to 8, characterized in that the selected strains are microorganisms which have the capacity of growing on the substrate(s), present in the medium, which is (are) formed during the fermentation method.

## Patentansprüche

1. Verfahren zur Kultivierung von Mikroorganismen, insbesondere von Hefen, auf Lactoserum als Ausgangssubstrat, dadurch gekennzeichnet, dass nach der Abtrennung der im Lactoserum enthaltenen Proteine und der Gewinnung der anderen Bestandteile die Kultivierung auf dem so erhaltenen Milchsäuresubstrat durchgeführt wird unter Verwendung einesteils eines Verbandes von Stämmen, von denen jeder auf spezielle Weise auf einem bestimmten in dem Milieu vorhandenen natürlichen Substrat oder auf den während des Fermentierungsprozesses gebildeten Metaboliten wächst und anderenteils bestimmter Mutanten eines Stammes, um ökologische Nischen zu bilden, die einem Verband von Stämmen entsprechen, die aus der gleichen Art hervorgehen.

2. Verfahren nach Anspruch 1, dadurch gekennzeichnet, dass der Verband der Stämme gebildet wird durch Kluyveromyces fragilis, das auf Lactose wächst, Kluyveromyces lactis, das auf Milchsäure wächst, Candida utilis und Saccharomyces cerevisae, die auf Glucose wachsen, sowie Torulopsis bovina, das auf Äthanol wächst.

3. Verfahren nach Anspruch 1, dadurch gekennzeichnet, dass das Ausgangssubstrat ein süsses oder saures Lactoserum ist, das von verschiedenen Milch- und Käsebehandlungen herrührt.

4. Verfahren nach Anspruch 3, dadurch gekennzeichnet, dass das Ausgangssubstrat teilweise oder vollständig durch Ausfällung mittels einer thermischen oder Säure-Behandlung, durch Ultrafiltrationsabtrennung oder Exklusionschromatographie sowie Ionenaustausch deproteinisiert ist.

5. Verfahren nach Anspruch 3, dadurch gekennzeichnet, dass das Ausgangssubstrat gleichfalls teilweise oder vollständig enzymatisch oder chemisch hydrolisiert ist.

6. Verfahren nach einem der Ansprüche 1 bis 5, dadurch gekennzeichnet, dass das Kulturmilieu von Haus aus aus einem Gemisch von zwei (oder mehr) Substraten besteht, die aus einer Gruppe ausgewählt werden, die aus Lactose, Milchsäure, Äthanol und gegebenenfalls Galactose und Glucose besteht.

7. Verfahren nach Anspruch 1, dadurch gekennzeichnet, dass im Inneren jeder der Nischen ein Wettbewerb zwischen den einzelnen Stämmen, die in der Lage sind, mit der grössten Ausbeute auf dem einzigen vorhandenen Substrat zu wachsen, aufrechterhalten wird.

8. Verfahren nach Anspruch 7, dadurch gekennzeichnet, dass die ausgewählten Stämme aus den Arten Kluyveromyces fragilis, Kluyveromyces lactis, Torulopsis bovina, Saccharomyces cerevisae oder Candida utilis hervorgehen.

9. Verfahren nach einem der Ansprüche 1 bis 8, dadurch gekennzeichnet, dass die ausgewählten Stämme Mikroorganismen sind, die in der Lage sind, auf dem oder den Substraten, die in dem Milieu vorhanden sind und die sich im Laufe des Fermentierungsprozesses bilden, zu wachsen.